# EUROPÄISCHE PATENTSCHRIFT

(11) **EP 0 923 600 B1**
(45) Veröffentlichungstag und Bekanntmachung des Hinweises auf die Patenterteilung: **21.06.2006**
(21) Anmeldenummer: 97936602.8
(22) Anmeldetag: 01.08.1997
(51) Int. Cl.: C07J 41/00, A61K 31/575, A61K 9/127, C12N 15/63, C07C 271/20, C07C 279/14, C07C 237/08, A61K 31/325, A61K 31/16, A61K 31/155

(54) **NEUARTIGE KATIONISCHE AMPHIPHILE FÜR DEN LIPOSOMALEN GENTRANSFER**
NOVEL CATIONIC AMPHIPHILIC LIPIDS FOR LIPOSOMAL GENE TRANSFER
AMPHIPHILES CATIONIQUES D'UN TYPE NOUVEAU UTILISES POUR LE TRANSFERT LIPOSOMAL DE GENES

(30) Priorität: 02.08.1996 DE 19631189
(43) Veröffentlichungstag der Anmeldung: 23.06.1999
(73) Patentinhaber: MAX-DELBRÜCK-CENTRUM FÜR MOLEKULARE MEDIZIN, 13125 Berlin (DE); SCHNEIDER, Manfred P., 42111 Wuppertal (DE)
(72) Erfinder: SCHNEIDER, Manfred, D-42111 Wuppertal (DE); KEIL, Oliver, D-42327 Wuppertal (DE); RESZKA, Regina, D-16341 Schwanebeck (DE); GROTH, Detlef, D-14548 Ferch (DE)
(74) Vertreter: Vogelsang-Wenke, Heike
(86) Internationale Anmeldenummer: PCT/DE1997/001669
(87) Internationale Veröffentlichungsnummer: WO 1998/005678

(56) Entgegenhaltungen:
- EP-A- 0 038 750
- WO-A-86/04579
- WO-A-94/04545
- WO-A-95/04030
- WO-A-96/14831
- WO-A-96/18372
- WO-A-96/22303
- WO-A-97/00241
- FR-A- 2 551 758
- US-A- 4 584 136

## Beschreibung

Die Erfindung betrifft kationische Lipide der allgemeinen Formel I, 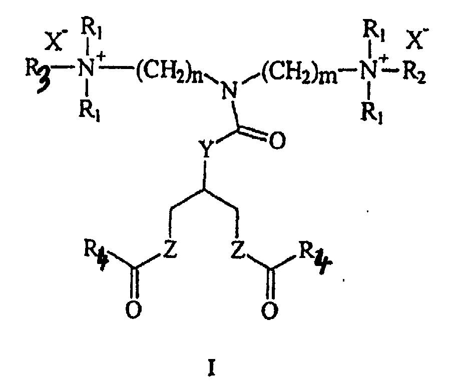 wobei n=2,3,4,6,8 und m=2,3,6,8 sein kann und R₁ = H, CH₃, CH₂CH₂OH; R₂ bzw. R₃ = H, CH₃, CH₂CH₂OH, (CH₂)₃N⁺(R₁)₃, R₄ einen geradkettigen, gesättigten oder ungesättigten aliphatischen Rest C₇-C₂₁, Z = CH₂, O, NH, Y = CH₂, O, NH und X = Cl, Br, I, CH₃COO, CF₃COO bedeutet.

Kationische Lipide der allgemeinen Formel I sind geeignete Reagenzien für den liposomalen Gentransfer (Transfektion). Anwendungsgebiete derartiger Transfektionsreagenzien liegen in der Medizin und in der Gentechnik.
Das Einbringen genetischen Materials in eukaryontische Zellen ist ein grundlegendes Verfahren zum Studium biologischer Funktionen und von zunehmender Bedeutung für die gentherapeutische Behandlung verschiedener Erkrankungen, von denen die Tumoren an erster Stelle zu nennen sind.
Man unterscheidet hierbei biologische, physikalische und physikochemische Methoden für den Transfer von DNA, RNA und Proteinen in die Zielzelle [Wagner J, Madry, H., Reszka, R (1995). *In vivo* gene transfer: focus on the kidney. Nephrol. Dial.Transptant10:1801-1807 Zhu J, Zhang L, Hanisch U-K, Felgner PL,. Reszka R (1996). In vivo gene therapy of experimental brain tumors by continuous administration of DNA-liposome complexes. Gene Therapy 3: 472-476, Kiehntopf, M., Brach MA & Hermann F (1995). Gentherapie in der Onkologie: Perspektiven, Chancen und Risiken. Onkologie 18 (Sonderheft): 16-26]. Physikalische Methoden wie Elektroporation und Mikroinjektion sind nur für den *ex vivo* und *in vitro* Transfer geeignet. Die sogenannte "Jet"- Injektions-Methode läßt sich darüberhinaus auch für den *in vivo* Gentransfer anwenden (Leber, Haut). Physikochemische Methoden wie Kalziumphosphatpräzipitationstechnik (KPP)- und DEAE-Dextran- Transfektion, sind auf *in vitro* und *ex vivo* Anwendungen begrenzt. Der zur Zeit in einer Reihe von klinischen Studien (Phase I/II) erprobte retrovirale Gentransfer mit Hilfe virusproduzierender Zellen ist durch eine längeranhaltende, aber relativ niedrige Genexpression in sich teilende Zellen gekennzeichnet. Problematisch beim retroviralen Gentransfer ist vor allem die Entwicklung einer spezifischen Immunantwort gegen die implantierten virusproduzierenden Helferzellen, die Möglichkeit der Generierung replikationskompetenter Viren und die Gefahr einer Aktivierung von zellulären Onkogenen bzw. Inaktivierung von Suppressorgenen als Resultat der zufälligen Lokalisation der Geninsertion. Die notwendigen umfangreichen zellbiologischen und medizinischen Vorarbeiten und die aufwendigen Sicherheitsvorkehrungen lassen hohe Kosten bei der klinischen Anwendung des retroviralen Gentransfers erwarten.
Vektoren auf der Basis von Adenoviren sind ebenfalls aussichtsreiche Gentransfervehikel. Diese erreichen hohe Transfektionsraten auch in sich nicht teilendem Gewebe. Da die DNA nicht in das Genom integriert wird, ist die Dauer der Fremdgenexpression begrenzt und einer wiederholten Anwendung *in vivo* steht die starke spezifische Immunantwort des Wirtsorganismus bei wiederholten Applikationen entgegen.
Der üposomal vermittelte Gentransfer hat dagegen in den letzten Jahren auch für *in vivo* Anwendungen an Bedeutung gewonnen. Die Genkonstrukte lassen sich entweder in die Liposomen verkapseln oder werden mit der Membran assoziiert. Liposomale Präparationen zeichnen sich durch eine einfache Handhabung, geringe Immunreaktivität und damit wiederholbare Anwendbarkeit bei niedrigem Sicherheitsrisiko für Anwender und "Empfänger" aus. Noch am Anfang steht die Anwendung von Immunoliposomen als Transportvehikel für genetisches Material.
Ein ebenfalls interessanter Ansatz ist die Verwendung fusogener Liposomen, die in ihrem Inneren einen Komplex aus DNA und Kemprotein (HMG I) tragen [Kaneda, Iwai, K., Uchida, T(1989). Increased expression of DNA cointroduced with nuclear protein in adult rat liver Science 243, 375-378,Kaneda Y, Kato, K., Nakanishi, M., Uchida, T.(1996) Introduction of plasmid DNA and nuclear protein into cells by using erythrocyte ghosts, liposomes, and Sendai virus. Methods-Enzymol. 221:317-327].
Seit einigen Jahren werden kationische Liposomen für den Transfer von DNA (Felgner PL, Gadek TR, Holm M et al. (1987). Lipofection: a highly efficient, lipid-mediated DNA transfection procedure. Proc Natl Acad Sci USA 84:7413-7417, Felgner JH, Kumar R, Sridhar CN et al. (1994). Enhanced gene delivery and mechanism studies with a novel series of cationic lipid formulations. J Biol Chem 269:2550-2561), Antisense-Oligomeren, Proteinen und Ribozymen mit Erfolg eingesetzt. DNA wird dabei über elektrostatische Wechselwirkungen mit der Membran der Liposomen assoziiert und über einen bisher noch unvollständig verstandenen Mechanismus in die Zielzelle transferiert. Die Transferrate *in vitro* ist dabei in Abhängigkeit von der Zellinie mit der Effzienz des retroviralen Gentransfer vergleichbar. Zur Zeit befinden sich die ersten kationischen Liposomen/DNA/Komplexe [DMRIE/DOPE und DOTMA/DOPE (Lipofectin, Gibco BRL USA) sowie DC-ChoUDOPE] in einer klinischen Phase I/II Prüfung (Gao, X., Huang, L. (1995) Cationic liposome-mediated gene transfer. Gene Therapy 2:710-722).
Kationische, amphiphile Moleküle, bestehend aus einem Lipidteil (Steroid-oder Diglycerid-Gerüst) und einer positiv geladenen Kopfgruppe (Ammonium-Ionen) sind in der Lage, entweder spontan oder nach Zusatz eines Hilfslipids (Dioleoylphosphatidylethanolamin (DOPE)) Liposomen auszubilden. DNA wird dabei über elektrostatische Wechselwirkungen mit der Membran der Liposomen assoziiert und über einen bisher noch unvollständig verstandenen Mechanismus in die Zielzelle transferiert.
Für einen möglichst effizienten in vitro und in vivo Gentransfer sollten die zur Generierung der Liposomen verwendeten kationischen Lipide die folgenden Kriterien erfüllen:
1. Sie sollten nicht toxisch und biologisch abbaubar sein und keine Immutueaktionen hervorrufen
2. Sie sollten möglichst effizient mit der DNA komplexieren, diese vor Abbaureaktionen schützen und hohe Transferraten aufweisen
3. Sie sollten Zellen rezeptorspezifisch transfizieren
4. Sie müssen leicht und in größeren Mengen synthetisiert werden können

WO 96/18372 offenbart. kationische Amphiphile, die zur Verwendung bei Transfektionsreaktionen geeignet sind.

Sowohl die Syntheseverfahren als auch die Moleküle selbst der bislang in der Literatur beschriebenen kationischen Amphiphile weisen eine Reihe von Nachteilen auf Die Synthese der kommerzialisierten Transfektionsreagenzien DOTMA (Lipofektin), DOGS und DOSPA verläuft über viele Stufen mit zum Teil sehr geringen Ausbeuten und die Aufreinigung der Produkte erfordert eine aufwendige säulenchromatographische Reinigung. Bei vielen Verbindungen wie z.B. DOTMA, DMRIE und DOSPA sind die kationischen Kopfgruppen mit langkettigen Fettalkoholen über Etherbindungen verknüpft, welche eine sehr schlecht biologische Abbaubarkeit und somit eine hohe Toxizität des Moleküls zur Folge haben.

Der Erfindung liegt die Aufgabe zu Grunde, neuartige kationische Lipide zu finden, welche im Vergleich mit den bislang bekannten Verbindungen höhere Crentransferraten und geringere Toxizüäten aufweisen. Weiterhin liegt der Erfindung die Aufgabe zu Grunde, Verfahren zu finden, welche die Synthese der kationischen Lipide in wenigen Reaktionsstufen und mit hohen Ausbeuten ermöglicht.
Diese Aufgaben wurde gemäß Anspruch 1 gelöst. Mit den neüartigen kationischen Lipiden der Formel I werden Verbindungen zur Verfügung gestellt, die den Anforderungen der Aufgabenstellung entsprechen.

Anspruch 2 definiert eine besonders bevorzeigte Ausführungs form der Erfindung.

Die Herstellung der erfindungsgemäßen Verbindungen erfolgt gemäß den beigefügten Schemata.

Schema I beschreibt das Verfahren zur Herstellung neuartiger kationischer Lipide mit 1,3-Diglycerid-Grundgerüst an denen die kationische Kopfgruppe über einen Carboxylat-Spacer angeknüpft ist.

Zusammenfassend kann festgestellt werden, das es nunmehr möglich ist, durch die beschriebenen Verfahren unter kostengünstigen Bedingungen und unter Verwendung preiswerter Chemikalien Transfektionsreagenzien zu synthetisieren, welche eine unerwartet hohe Gentransferrate und eine gute biologische Abbaubarkeit aufweisen.

Die Erfindung soll nachfolgend durch Ausführungsbeispiele näher erläutert werden.

### Ausführungsbeispiele

### Beispiel 1

### Herstellung von 2-(N⁵-carbamoyl-(N¹,N¹⁰,N¹⁴-ammoniumtetraazadecan))-1,3-dioleoyloxy-propan-tri-trifluoracetat I

247 µl (1 mmol) 1,3-Dibenzyloxy-2-propanol wurden mit 7 ml einer 20%igen Lösung von Phosgen in Toluol versetzt und 48 h bei RT gerührt. Nach dieser Zeit wurde überschüssiges Phosgen mit Argon ausgetrieben und der Ansatz zu einer Lösung von 302 mg (0.6 mmol) *N*¹,*N*¹⁰,*N*¹⁴-tris-BOC-spermin und 300 µl Triethylamin in 5 ml Methylenchlorid zugetropft und für 4 h bei RT gerührt. Anschließende Säulenchromatographische Reinigung (Kieselgel 60) ergaben 375 mg (47%) 2-(*N*⁵-carbamoyl-(*N*¹,*N*¹⁰,*N*¹⁴-tris-BOC-tetraazadecan))-1,3-dibenzyloxy-propanol (zähes Öl).
670 mg (0.84 mmol) 2-(*N*^{*5*}-carbamoyl-(*N*^{*1*},*N*^{*10*},*N*^{*14*}-tris-BOC-tetraazadecan))-1,3-dibenzyloxy-propanol wurden daraufhin in 25 ml Ethanol gelöst, mit 150 mg Pd/C (5%) versetzt und 24 h hydriert. Nach Absaugen des Katalysators erhielt man 520 mg (100%) 2-(*N*^{*5*}*-*carbarmoyl-(*N*^{*1*},*N*^{*10*},*N*^{*14*}-tris-BOC-tetraazadecan))-1,3-propantriol (zähes Öl).
472 mg (0.76 mmol) 2-(*N*^{*5*}-carbamoyl-(*N*^{*1*},*N*^{*10*},*N*^{*14*}-tris-BOC-tetraazadecan))-1,3-propantriol wurden mit 452 mg (1.6 mmol) Ölsäure, 334 mg (1.62 mmol) DCC und 20 mg DMAP in 30 ml Methylenchlorid gelöst und 24 h bei RT gerührt. Anschließende Säulenchromatographische Reinigung (Kieselgel 60) ergaben 457 mg (52%) 2-(*N*^{*5-*}carbamoyl-(*N*¹,*N*¹⁰,*N*¹⁴-tris-BOC-tetraazadecan))-1,3-dioleoyloxy-propan (zähes Öl).
200 mg (0.17 mmol) 2-(*N*⁵-carbamoyl-(*N*¹,*N*¹⁰,*N*¹⁴-tris-BOC-tetraazadecan))-1,3-dioleoyloxy-propan wurden mit 3 ml TFA/CH₂Cl₂ (1:1) versetzt und 30 min bei RT gerührt. Der Ansatz wurde mit 10 ml Dioxan verdünnt und im Hochvakuum vom Lösungsmittel befreit. Man erhielt 202 mg (99%) 2-(*N*⁵-carbamoyl-(*N*¹,*N*¹⁰,*N*¹⁴-ammoniumtetraazadecan))-1,3-dioleoyloxy-propan-tri-trifluoracetat I (zähes, farbloses Öl).

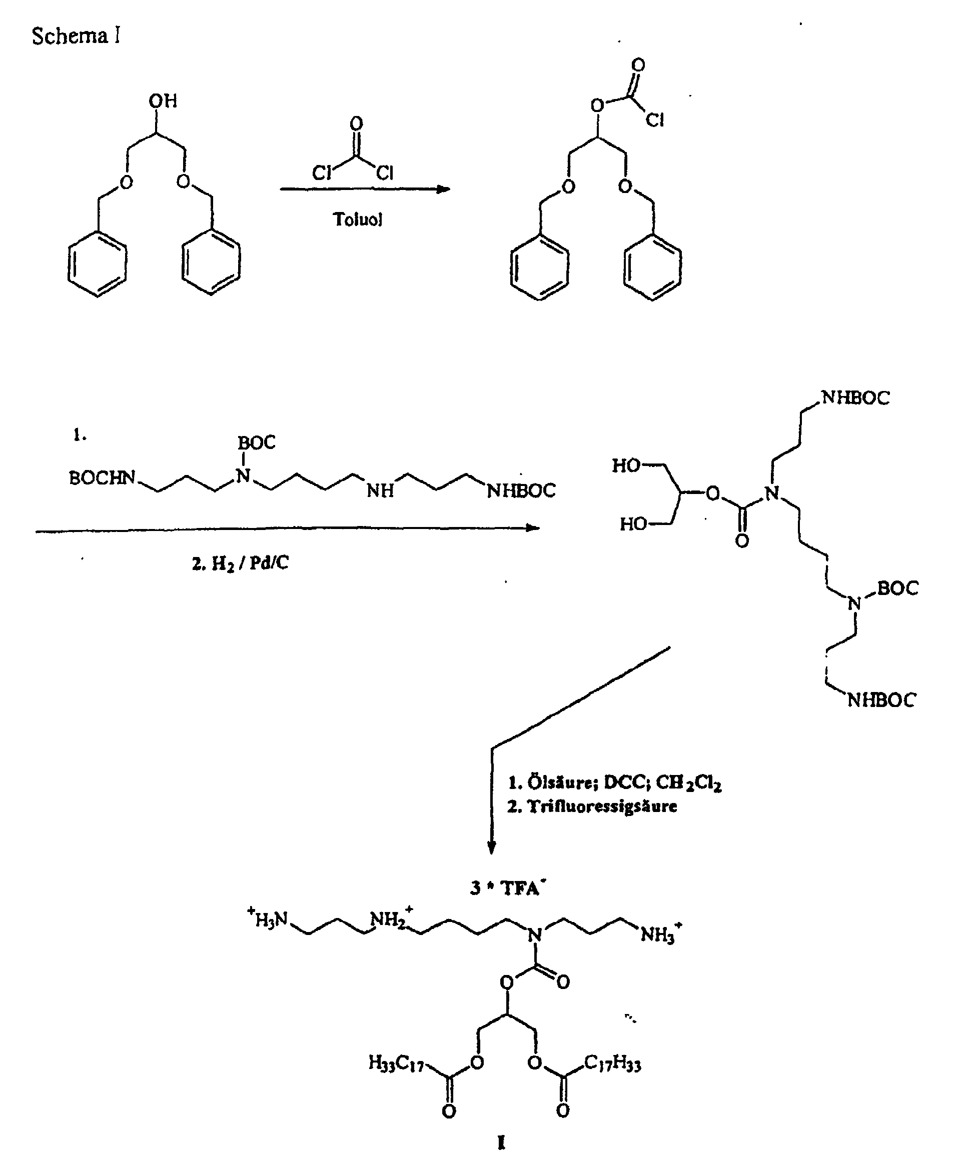

## Patentansprüche

1. Kationische Lipide der allgemeinen Formel I 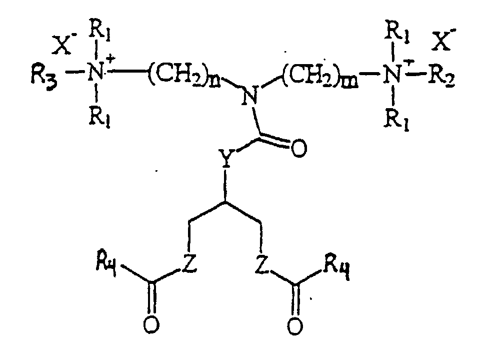 wobei
n = 2, 3, 4, 6 oder 8
m = 2, 3, 6, oder 8
R₁ = H, CH₃ oder CH₂CH₂OH
R₂ = H, CH₃, CH₂CH₂OH oder (CH₂)₃N⁺(R₁)₃
R₃ = H, CH₃, CH₂CH₂OH oder (CH₂)₃N⁺(R₁)₃
R₄ = geradkettiger, gesättigter oder ungesättigter aliphatischer Rest C₇ - C₂₁
Z = CH₂, **O** oder NH
Y = CH₂, O oder NH
X = Cl, Br, I, CH₃COO , CF₃COO

2. Lipid nach Anspruch 1, **dadurch gekennzeichnet, dass** es DOCSPER mit der folgenden Formel ist, 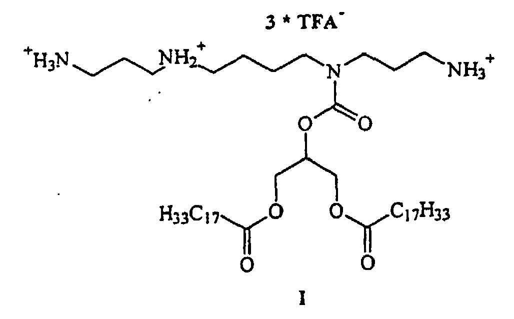 wobei
n =4
m =3
R₁ = H,
R₂ = H,
R₃ = (CH₂)₃N⁺H₃
R₄ = C₁₇H₃₃ (Ölsäure)
Z = O
Y =O
X = CF₃COO⁻

## Claims

1. Cationic lipids of the general formula I 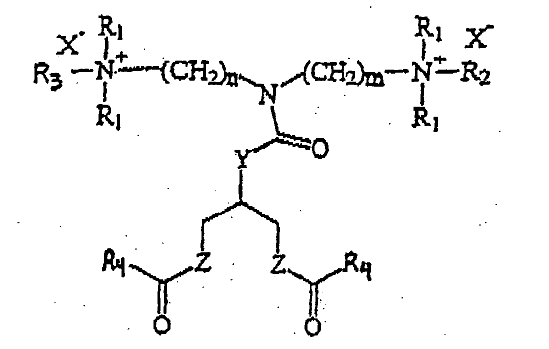 wherein
n = 2, 3, 4, 6 or 8
m = 2, 3, 6 or 8
R₁ = H, CH₃ or CH₂CH₂OH
R₂ = H, CH₃, CH₂CH₂OH or (CH₂)₃N⁺(R₁)₃
R₃ = H, CH₃, CH₂CH₂OH or (CH₂)₃N⁺(R₁)₃
R₄ = a straight-chain, saturated or unsaturated C₇-C₂₁ aliphatic radical
Z = CH₂, O or NH
Y = CH₂, O or NH
X = Cl, Br, I, CH₃COO, CF₃COO

2. Lipid according to claim 1, **characterised in that** it is DOCSPER having the following formula 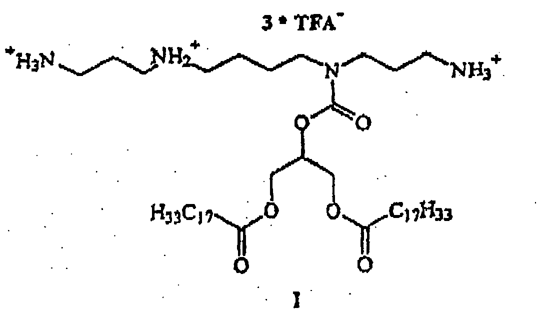 wherein
n = 4
m = 3
R₁ = H
R₂ = H
R₃ = (CH₂) ₃N⁺H₃
R₄ = C₁₇H₃₃ (oleic acid)
Z = O
Y = O
X = CF₃COO⁻

## Revendications

1. Lipides cationiques de formule générale I 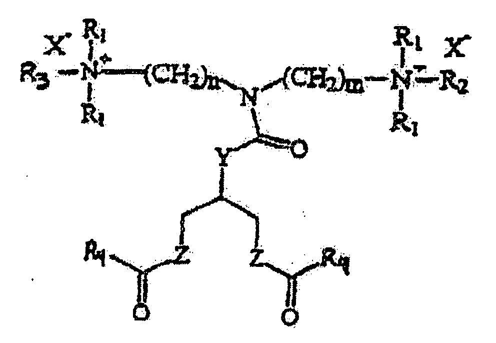 dans laquelle
n = 2, 3, 4, 6 ou 8
m = 2, 3, 6 ou 8
R₁= H, CH₃ ou CH₂CH₂OH
R₂= H, CH₃, CH₂CH₂OH ou (CH₂) ₃N⁺(R₁)₃
R₃ = H, CH₃, CH₂CH₂OH ou (CH₂)₃N⁺(R₁)₃
R₄ = un reste aliphatique en C₇-C₂₁ à chaîne linéaire, saturé ou insaturé,
Z = CH₂, O ou NH
Y= CH₂, O ou NH
X= Cl, Br, I, CH₃COO, CF₃COO.

2. Lipide selon la revendication 1, **caractérisé en ce qu'**il est DOCSPER ayant la formule suivante 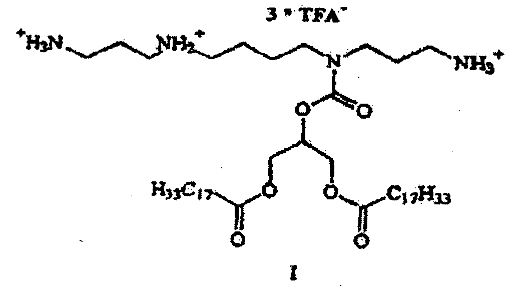 dans laquelle
n = 4
m = 3
R₁ = H
R_{2 =} H
R₃ = (CH₂)₃N⁺H₃
R₄ = C₁₇H₃₃ (acide oléique)
Z = O
Y = 0
X = CF₃COO⁻
